# EUROPEAN PATENT APPLICATION

(11) **EP 4 427 745 A1**
(43) Date of publication of application: **11.09.2024**
(21) Application number: 22889510.8
(22) Date of filing: 04.11.2022
(51) Int. Cl.: A61K 31/16, A61L 2/16

(54) **COMPOSITION WITH VIRUCIDAL ACTIVITY, USE AND APPLICATION METHOD THEREOF**

(30) Priority: 05.11.2021 ES 202131039
(71) Applicant: Bio Logic Crop Science, S.L., 46010 Valencia (ES)
(72) Inventor: CASAÑA GINER, Victor, 46010 Valencia (ES)
(74) Representative: Soler Lerma, Santiago
(86) International application number: PCT/ES2022/070713
(87) International publication number: WO 2023/079206

(57) **Abstract**

Increasingly, infectious diseases due to viruses, bacteria, fungi and other contact-transmissible microorganisms, droplets and/or aerosols, are acquiring greater importance due to globalisation and the ease of dissemination between very distant countries. A characteristic example is the Influenza A pandemic in 2009 and the SARS pandemics due to the SARS-Cov virus in 2003 and the COVID-19 pandemic due to the SARS-CoV-2 virus in 2020. To combat infections that may occur by contact, or by aerosolised drops (including those surfaces contaminated with infectious agents), a wider palette of antimicrobials and virucides is required. The present invention resolves this problem for viral infections with the use of a low toxicity surfactant from the group of trimethylammonium methyl sulfates.

## Description

As the name suggests, the present invention relates to compounds with virucidal activity, in particular comprising low toxicity amide methyl sulfate virucides active against coronaviruses, influenza viruses and non-lipid enveloped viruses.

One of the objectives of the present invention is its utility at the industrial and regulatory level to tackle situations of need against infectious agents quickly, safely and in accordance with the strict legal requirements for biocides.

### BACKGROUND

The EPA (Environmental Protection Agency, USA) has authorised the following active ingredients against human coronaviruses and in particular against COVID-19 (it being understood hereinafter that COVID-19 is the syndrome - the disease - and SARS-CoV-2 the virus that causes the COVID-19 syndrome; from the context it is simply deduced whether it refers to the virus or the syndrome or both, for example, against COVID-19, it implies against SARS-CoV-2 and implicitly, against COVID-19).

Compounds declared active against COVID-19 by the EPA are:
- 1,2-Hexanediol
- Chlorine dioxide
- Chlorine dioxide; Quaternary ammonium
- Citric acid
- Citric acid; Thymol
- Dodecylbenzenesulfonic acid
- Dodecylbenzenesulfonic acid; L-Lactic acid
- Ethanol
- Ethanol (Ethyl alcohol); Phenolic
- Glycolic acid
- Hydrochloric acid
- Hydrogen chloride
- Hydrogen peroxide
- Hydrogen peroxide; Ammonium carbonate; Ammonium bicarbonate
- Hydrogen peroxide; Octanoic acid; Peroxyacetic acid (Peracetic acid)
- Hydrogen peroxide; Peroxyacetic acid (Peracetic acid); Octanoic acid
- Hydrogen peroxide; Peroxyoctanoic acid; Octanoic acid
- Hydrogen peroxide; Peroxyoctanoic acid; Peroxyacetic acid (Peracetic acid)
- Hydrogen peroxide; Quaternary ammonium
- Hydrogen peroxide; Silver
- Hypochlorous acid
- Iodine
- Isopropanol
- Isopropanol; Phenolic
- Isopropanol; Quaternary ammonium
- L-Lactic acid
- Octanoic acid
- Peroxyacetic acid (Peracetic acid)
- Peroxyacetic acid; Hydrogen peroxide
- Phenolic
- Phenolic; Ethanol (Ethyl alcohol)
- Polyhexamethylene biguanide; Quaternary ammonium
- Potassium peroxymonosulfate; Sodium chloride
- Quaternary Ammonium
- Silver
- Silver ion; Citric acid
- Sodium carbonate peroxyhydrate; Tetraacetyl ethylenediamine
- Sodium chloride
- Sodium chlorite
- Sodium chlorite; Citric acid
- Sodium chlorite; Sodium dichloroisocyanurate dihydrate
- Sodium dichloroisocyanurate
- Sodium hypochlorite
- Sodium hypochlorite; Sodium carbonate
- Tetraacetyl ethylenediamine
- Thymol
- Triethylene glycol; Quaternary ammonium

Among the known virucidal compounds, the following are non-extensive of all the compounds described to date:
- PH modifiers
- Peroxides and derivatives
- Metals
- Iodates and derivatives
- Chlorinates and derivatives
- Brominates and derivatives
- Terpenes
- Fat-soluble cations
- Salts
- Short-chain alcohols (number of carbons fewer than or equal to four)
- Phenols
- Short-chain aldehydes (number of carbons fewer than or equal to four)
- Tertiary amines

Within the fat-soluble cations, there are two relevant groups, mainly biguanide derivatives and quaternary ammonium compounds.

Among the quaternary ammonium compounds, the following are described with virucidal activity, among others:
- Tetradecyl dimethylbenzyl ammonium chloride {TDBAC}
- Alkyl dimethyl benzylammonium chlorides (wherein the alkyl group is chosen from dodecanyl, tetradecanyl and/or hexadecanyl)
- Alkyl dimethylethyl benzylammonium chlorides (wherein the alkyl group is chosen from dodecanyl and/or tetradecanyl) {ADEBAC}
- Didecyldimethylammonium chloride
- N, N-dimethyl-N-octyl-1-octanaminium chloride
- N-decyl-N, N-dimethyl-1-decanaminium chloride
- N, N, N-trimethyl-1-hexadacanaminium chloride
- N,N-Dimethyl-N-[3-(trimethoxysilyl)propyl] -1-octadecanaminium chloride
- Poly(iminoimidocarbonyliminoimidocarbonylimidohexamethylene hydrochloride)

As far as quaternary ammonium compounds are concerned, it is notable that not all of them have been described as virucidal, some even only show virucidal activity against viruses enveloped with a lipid layer. Nor is the use appreciated of methyl sulfates of quaternary ammonium compounds whose lipid chains have an unsaturation.

As can be seen, there are a multitude of chemical groups that have a virucidal contact activity; nevertheless, most of these have toxicity problems, especially by inhalation and also ocular. A problem of the State of the Art is to find virucides applied by means of techniques that micronise the droplets containing the virucides (such as those generated in aerosol guns or pressurised aerosols) that do not have high toxicity by inhalation for mammals, are very dangerous ocular corrosives and are also biodegradable. It should be noted that the virucides used on surfaces can be in contact with skin, food and other utensils that can end up being a route of entry into the human body.

In the following, given the complexity of the compounds and the very diverse forms of nomenclature they have (EINECS, CAS, IUPAC, trade names, etc.), the Chem to Name system will be used in the terminology of the compounds described in the present invention as described in "Name=Struct: A Practical Approach to the Sorry State of Real-Life Chemical Nomenclature" (J. Brecher, 1999, J. Chem. Inf. Comput. Sci. 1999, 39, 943-950; DOI: 10.1021/ci990062c), as adopted by various computer packages such as ChemBioOffice^{®} from Cambridge Software and others.

US 6,207,241 discloses methyltrimethyl- [3-(undec-10-enoylamino)propyl]azanium sulfate as a component of an antifungal shampoo, combined with an ergosterol synthesis-inhibiting fungicide. However, its effect is not that of a fungicide per se, but that of synergising the effect of the aforementioned ergosterol synthesis inhibitor fungicide or methyltrimethyl- [3-(undec-10-enoylamino)propyl]azanium sulfate itself, against Malassezia furfur (Pytirosporum ovale), Epidermophyton, Microsporum and Trychophyton. Industrially, US 6,207,241 discloses that the compound is present in ca. 40-45% in Rewocid UTM 185. At today's date, Evonik presents the compound as discontinued and as used in shampoos, anti-dandruff shampoos, liquid soaps, masks and creams for the skin and lotions, at a usage ratio of 1 to 3%. However, it neither mentions nor suggests its use as a virucide.

Additionally, the product Tetranyl U from Kao Chemicals, describes methyltrimethyl- [3-(undec-10-enoylamino)propyl]azanium sulfate in its Technical Data Sheet as a cationic surfactant with a known fungicidal effect on *Candida albicans, Aspergillus niger,* and with a bactericidal effect against *Staphylococcus aureus, Pseudomonas aeruginosa* and *Escherichia coli.*

The use according to ECHA's ongoing registration of the compounds of the present invention (for example, the compound with test code BLCS-19)is in cosmetics and personal hygiene, as well as to be incorporated into washing and cleaning products, there being no mention of its virucidal properties.

### DESCRIPTION OF THE INVENTION

The present invention is an alternative solution to the problem of using virucides, overcoming the aforementioned drawbacks, based on the use of unsaturated N, N, N-trimethylpropan-1-aminium or N, N, N-dimethylethyl-1-aminium amide methyl sulfate compounds.

In particular, the following: 3-(undec-10-enamido)-N,N, N-trimethyl-propan-1-aminium methyl sulfate, 3-(dodec-11-enamido)-N,N,N-trimethylpropan-1-aminium methyl sulfate, 3-(undec-10-enamido)-N-ethyl-N,N-dimethylpropan-1-aminium methyl sulfate, and 3-(dodec-11-enamido)-N-ethyl-N,N-dimethylpropan-1-aminium methyl sulfate.

From the tests carried out, it is obtained that they are virucidal compounds of viruses both with and without lipid envelope. Therefore, they can be applied for disinfection used at a percentage that is easy to deduce for the person skilled in the art based on the observation of the effective amount to resolve the possible differences between sensitivity of the viruses against the compounds of the present invention.

That is, the viruses for which the compounds of the present invention can be applied are:

**Table 1. Virucides according to the present invention**

| Test code: BLCS-19 |
|---|
| 3-(Undec-10-enamido)-N,N, N-trimethyl-propan-1-aminium methyl sulfate |
| Chemical formula: C18H38N205S |
| Exact mass: 394.25 a.m.u. |
| Molecular mass: 394.57 a.m.u. |
| Elemental analysis: C, 54.79%; H, 9.71%; N, 7.10%; O, 20.27%; S, 8.13% |
| 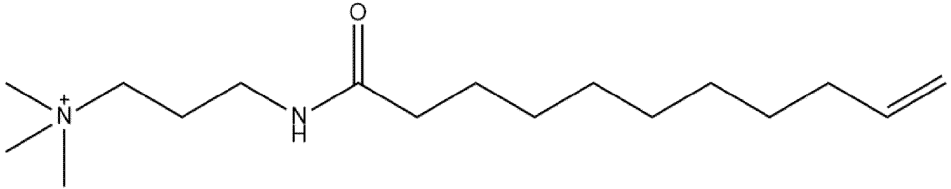 |
| 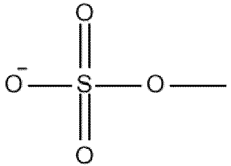 |

| Test code: BLCS-20 |
|---|
| 3-(Dodec-11-enamido)-N,N, N-trimethylpropan-1-aminium methyl sulfate |
| Chemical formula: C19H40N2O5S |
| Exact mass: 408.27 a.m.u. |
| Molecular mass: 408.60 a.m.u. |
| Elemental analysis: C, 55.85; H, 9.87; N, 6.86; O, 19.58; S, 7.85 |
| 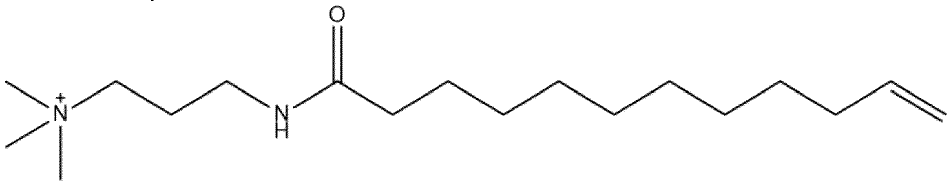 |
| 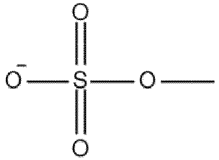 |

| Test code: BLCS-21 |
|---|
| 3-(Undec-10-enamido)-N-ethyl-N,N-dimethylpropan-1-aminium methyl sulfate |
| Chemical formula: C19H40N2O5S |
| Exact mass: 408.27 a.m.u. |
| Molecular mass: 408.60 a.m.u. |
| Elemental analysis: C, 55.85; H, 9.87; N, 6.86; O, 19.58; S, 7.85 |
| 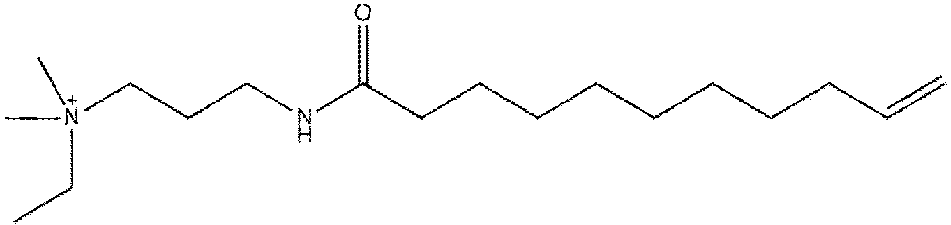 |
| 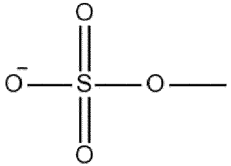 |

| Test code: BLCS-22 |
|---|
| 3-(Dodec-11-enamido)-N-ethyl-N,N-dimethylpropan-1-aminium methyl sulfate |
| Chemical formula: C20H42N2O5S |
| Exact mass: 422.28 |
| Molecular mass: 422.62 |
| Elemental analysis: C, 56.84%; H, 10.02%; N, 6.63%; O, 18.93%; S, 7.59% |
| 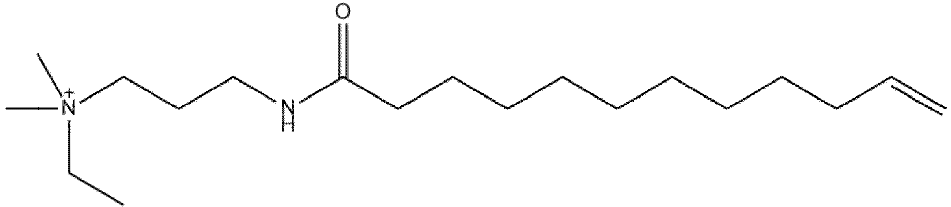 |
| 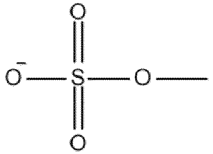 |

### DESCRIPTION OF EMBODIMENTS

### Example 1.

The following formulations are made according to the present invention and with the indicated compositions:

| | |
|---|---|
| Formula 1-1 | |

| | Concentration w/w |
|---|---|
| Water | 99.75% |
| 3-(Undec-10-enamido)-N,N,N-trimethyl-propan-1-aminium methyl sulfate | 0.25% |

| Formula 1-2 | Concentration w/w |
|---|---|
| Ethanol | 70% |
| Propylene glycol | 29.75% |
| 3-(Undec-10-enamido)-N,N,N-trimethyl-propan-1-aminium methyl sulfate | 0.25% |

The assay (equally declared as a 70% ethanol solution, to avoid possible bias in the analysis) of its virucidal activity is carried out according to standard NF EN 14476:2013 + A2:2019, on poliovirus type 1, adenovirus type 5, murine norovirus and coronavirus 229E.

The experimental conditions are the following:

| | | |
|---|---|---|
| • Temperature: | | 37°C ± 1°C |
| • Titration method | TCID50 (50% infective dose for cell culture) | |
| • Concentrations: | | 80%; 50%; 0.1 %. |
| • Contact times: | 5 minutes. | |
| • Contact temperature | | 30 ºC ± 1 ºC |
| • Diluent used | Sterile distilled water | |
| • Appearance of product dilutions | Transparent. | |

- Interfering substances:
   Clean conditions in the presence of bovine serum albumin 0.3 g/L.
- Method to eliminate cytotoxicity: Molecular filtration.
- Method to stop action: Cooling with ice.

The reduction with the 95% confidence interval for each formulation and virus is shown in the following results table 1.

| | Conc. | log reduction | | | Conc. | log reduction | | |
|---|---|---|---|---|---|---|---|---|
| Poliovirus type 1 ATCC VR-192 | 80% | 4.83 | + | 0.50 | 80% | 4.92 | + | 0.48 |
| Adenovirus type 5 ATCC VR-5 | 80% | 5.49 | + | 0.34 | 80% | 5.66 | + | 0.34 |
| Murine norovirus S99 Berlin | 80% | 5.91 | + | 0.34 | 80% | 6.8 | + | 0.35 |
| | 50% | 4.17 | + | 0.05 | 50% | 4.26 | + | 0.50 |
| Coronavirus 229E ATCC VR-740 | 50% | >6 | + | 0.37 | 80% | >6 | + | 0.35 |
| Influenza A H1N1 pdm09 A/California/7/2009 | 50% | 5.83 | + | 0.40 | 80% | 5.75 | + | 0.40 |

It can be seen that the efficacy is similar both in the presence of ethanol, which is virucidal to some extent per se, for lipid-enveloped viruses (but not without lipid envelope), and in the presence of water.

The enhancement of the virucidal effect is remarkable if the composition contains ethanol and propylene glycol in the case of norovirus, with a reduction of ca. 1 log.

Said virucidal solutions are used according to the application and circumstances in concentrations of 0.01% to 5%, regardless of the liquid medium selected.

### Example 2.

Alternative formulations to those tested in Example 1.

| Formula 2-1 | |
|---|---|
| | Concentration w/w |
| Water | 99.75% |
| 3-(Dodec-11-enamido)-N,N,N-trimethylpropan-1-aminium methyl sulfate | 0.25% |

| Formula 2-2 | |
|---|---|
| | Concentration w/w |
| Water | 99.75% |
| 3-(Undec-10-enamido)-N-ethyl-N,N-dimethylpropan-1-aminium methyl sulfate | 0.25% |

| Formula 2-3 | |
|---|---|
| | Concentration w/w |
| Water | 99.75% |
| 3-(Dodec-11-enamido)-N-ethyl-N,N-dimethylpropan-1-aminium methyl sulfate | 0.25% |

| Formula 2-4 | |
|---|---|
| | Concentration w/w |
| Water | 50% |
| Isopropanol | 20% |
| Ethanol | 20 % |
| 3-(Dodec-11-enamido)-N,N,N-trimethylpropan-1-aminium methyl sulfate | 5% |
| Benzalkonium chloride | 3% |
| Povidone | 1% |

| Formula 2-5 | |
|---|---|
| | Concentration w/w |
| Ethanol | 70 % |
| Propylene glycol | 29.75% |
| 3-(Undec-10-enamido)-N,N,N-trimethyl-propan-1-aminium methyl sulfate | 0.25% |

| Formula 2-6 | |
|---|---|
| | Concentration w/w |
| Ethanol | 70 % |
| Propylene glycol | 29.75% |
| 3-(Undec-10-enamido)-N-ethyl-N,N-dimethylpropan-1-aminium methyl sulfate | 0.25% |

| Formula 2-7 | |
|---|---|
| | Concentration w/w |
| Ethanol | 70% |
| Propylene glycol | 29.75% |
| 3-(Dodec-11-enamido)-N-ethyl-N,N-dimethylpropan-1-aminium methyl sulfate | 0.25% |

| Formula 2-8 | |
|---|---|
| Concentration w/w | |
| Ethanol | 70% |
| Propylene glycol | 29.75% |
| 3-(Dodec-11-enamido)-N-ethyl-N,N-dimethylpropan-1-aminium methyl sulfate | 0.25% |

The compounds of the present invention can be used at any concentration in which they show virucidal activity in any medium of presentation and application (be it gas, liquid or solid) and considering the contact time, the concentration of 0.01% to 5% w/w being preferred, preferably between 0.1% to 0.3% w/w, in water or in a mixture of 70% w/w ethanol and propylene glycol. These solutions can be formulated, placed in pressurised aerosol-generating containers or unpressurised spray guns, to then be sprayed.

As we can see, the compounds of the present invention can be used effectively against lipid enveloped viruses and non-lipid enveloped viruses.

These viruses, in their preferred nomenclature, are: Adeno-associated virus, Aichi virus, Australian bat lyssavirus, Astrovirus MLB1, Astrovirus VA1, BK polyomavirus, Banna virus, Barmah forest virus, Bundingo ebolavirus, Bunyamwera virus, Bunyavirus La Crosse, Bunyavirus snowshoe hare, Cercopithecine herpesvirus, Chandipura virus, Chikungunya virus, Cosavirus A, Cowpox virus, Coxsackievirus, Crimean-Congo hemorrhagic fever virus, Dengue virus, Dhori virus, Dugbe virus, Duvenhage virus, Eastern equine encephalitis virus, Ebolavirus, Echovirus, Encephalomyocarditis virus, Epstein-Barr virus, European bat lyssavirus, GB virus C, Hepatitis G virus, Hantaan virus, Hendra virus, Hepatitis A virus, Hepatitis B virus, Hepatitis C virus, Hepatitis E virus, Hepatitis delta virus, Horsepox virus, Human adenovirus, Human astrovirus, Human bocavirus, Human bocavirus 2, Human coronavirus (including SARS-CoV-1, SARS-CoV-2, MERS, HKU1, E229, OC43, NL63), Human cosaviruses A, B, C, D, E, Human cytomegalovirus, Human enterovirus 68, Human enterovirus 70, Human herpesvirus 1, Human herpesvirus 2, Human herpesvirus 6, Human herpesvirus 7, Human herpesvirus 8, Human immunodeficiency virus, Human papillomavirus 1, Human papillomavirus 2, Human papillomavirus 16, Human papillomavirus 18, Human papillomavirus 116, Human parainfluenza, Human parvovirus 4, Human parvovirus B19, Human respiratory syncytial virus, Human rhinovirus, Human SARS coronavirus, Human spumaretrovirus, Human T-lymphotropic virus, Human torovirus, Influenza A virus, Influenza B virus, Influenza C virus, Influenza A(H1N1)pdm09 virus, Influenza A(H3N2) viruses, Influenza A(H5N1) virus Isfahan virus, JC polyomavirus, Japanese encephalitis virus, Junin arenavirus, KI Polyomavirus, Klassevirus, Kunjin virus, Lagos bat virus, Lake Victoria Marburgvirus, Langat virus, Lassa virus, Lordsdale virus, Louping ill virus, Lymphocytic choriomeningitis virus, Machupo virus, Mayaro virus, Measles virus, Melaka virus, Mengo encephalomyocarditis virus, Merkel cell polyomavirus, Mokola virus, Molluscum contagiosum virus, Monkeypox virus, Mumps virus, Murray valley encephalitis virus, New York virus, Nipah virus, Norwalk virus, O'nyong-nyong virus, Orf virus, Oropouche virus, Pichinde virus, Poliovirus, Punta toro phlebovirus, Puumala virus, Rabies virus, Rift valley fever virus, Rosavirus A, Ross river virus, Rotavirus A, Rotavirus B, Rotavirus C, Rubella virus, Sagiyama virus, Salivirus A, Sandfly fever Sicilian virus, Sapporo virus, Semliki forest virus, Seoul virus, Simian foamy virus, Simian virus 5, Sindbis virus, Southampton virus, St. louis encephalitis virus, Tick-borne powassan virus, Torque teno virus, Toscana virus, Uukuniemi virus, Vaccinia virus, Varicella-zoster virus, Variola virus, Venezuelan equine encephalitis virus, Vesicular stomatitis virus, Western equine encephalitis virus, WU polyomavirus, West Nile virus, Yaba monkey tumor virus, Yaba-like disease virus, Yellow fever virus, Zika virus.zz

Of particular interest is the use of the compounds of the invention for disinfection of the following viruses:
HPV (human papilloma), HIV (AIDS), Herpes simplex, Herpes Varicella-Zoster (HZV), Hepatitis A, B, C, delta, E, adenovirus, coronavirus (in particular SARS and SARS-CoV-2), normovirus, coxackievirus.

The compounds of the present invention can be mixed with other known antiseptics to increase biocidal efficacy, both synergistically and by addition of effects.

In particular, they can be combined, at the doses known from the state of the art, with: 3,6-Diamino-10-methylacridinium Chloride; 8-Hydroxyquinoline product; 8-Hydroxyquinoline sulfate ointment; Acriflavine product; Acrisorcin; Actinoquinol; Alexidine; Aluminum acetate; Ambazone; Aminacrine hydrochloride; Aminacrine product; Aminacrine pyruvate; Aminacrine undecylate; Amphomycin calcium; Amylmetacresol; Auriclosene; Azelaic acid; Bensalan; Benzalkonium product; Benzethonium chloride; Benzethonium product; Benzododecinium; Benzododecinium bromide; Benzoin; Benzoxiquine; Benzoxonium chloride; Benzoyl peroxide; Benzyl alcohol; Benzylsulfamide; Bibrocathol; Biclotymol; Bismuth subgallate; Bismuth tribromophenate; Boric acid; Calcium hydroxide; Camphor product; Carbamide peroxide; Cetalkonium; Cetalkonium chloride; Cetrimonium; Cetrimonium chloride; Cetylpyridinium acetate; Cetylpyridinium bromide; Cetylpyridinium chloride; Cetylpyridinium chloride anhydrous; Cetylpyridinium product; Chaulmosulfone; Chloramine-T; Chlorhexidine gluconate; Chlorhexidine hydrochloride; Chlorhexidine product; Chlorindanol; Chloroazodin; Chlorocresol; Chlorothymol; Chloroxylenol; Chlorphenesin product; Chlorphenoctium amsonate; Cicliomenol; Cinoquidox; Cloflucarban; Cloguanamil; Cloponone; Cocamidopropylbetaine (in water); Crotamiton; Crotoniazide; Cuprimyxin; Cyclomenol; Dakins solution; Debropol; Decominol; Deditonium; Deditonium bromide; Dibromopropamidine product; Dichlorobenzyl alcohol; Dichloroxylenol; Dicresulene; Dipyrithione; Disiquonium; Disiquonium chloride; Dodeclonium bromide; Dofamium; Dofamium chloride; Famiraprinium chloride; Fenticlor product; Fepradinol; Fludazonium; Fludazonium chloride; Fluorosalan; Furmethoxadone; Gloxazone; Guaiacol product; Halazone; Halopenium chloride; Haloprogin; Hexachlorophene product; Hexedine; Hexetidine product; Hexylresorcinol product; Hydrogen peroxide; Iodoform; Laurcetium; Laurcetium bromide; Laurixamine; Lauroguadine; Laurolinium acetate; Lopobutan; Mafenide acetate; Mecetronium; Mecetronium ethylsulfate; Mequidox; Meralein; Merbromin product; Mercurous iodide; Mercury ammonium chloride product; Metabromsalan; Methaniazide; Methylisothiazolinone; Monalazone; Monalazone disodium; Morniflumate; Myristalkonium Chloride; Nibroxane; Nifuradene; Nifuraldezone; Nifuralide; Nifurethazone; Nifurfoline; Nifurimide; Nifurizone; Nifuroquine; Nifuroxazide; Nifuroxime; Nifurpirinol; Nifurprazine; Nifurvidine; Nifurzide; Nitromersol; Noxythiolin product; Octafonium chloride; Octenidine saccharin; Olanexidine; Opratonium iodide; Oxyquinoline sulfate; Parachlorophenol; penoctonium; Penoctonium bromide; Phanquone; Phenolate sodium; Phenoxyethanol product; Phenyl ethyl alcohol; Phthalylsulfacetamide; Picloxydine; Picric acid; Pirralkonium; Pirralkonium bromide; Pirtenidine; Pirtenidine hydrochloride; Prednicarbate; Proflavine dihydrochloride; Proflavine hemisulfate; Proflavine product; Proflavine sulfate; Resorcinol; Resorcinol monoacetate; Romifenone; Salicylanilide; Sanguinarium chloride; Selenium sulfide; Sepazonium; Sepazonium chloride; Silver nitrate; Silver oxide; Silver picrate; Silver sulfadiazine; Sodium meralein; Sulfacecole; Sulfarsphenamine; Symclosene; Talmetoprim; Tavaborole; Temodox; Tetradonium; Tetradonium bromide; Thimerosal product; Thymol iodide; Thymol product; Tibezonium; Tibezonium iodide; Toliodium; Toliodium chloride; Tribromsalan; Trichlorocarbanilide product; Undecoylium chloride; Zinc carbonate; Zinc phenolsulfonate;

To the extent the compounds of the present invention can be applied in vivo, the present invention likewise finds application as a topical and/or systemic antiviral in medical and veterinary applications. There is no reason why compounds should not exert their virucidal and antiviral activity in vivo, especially noting that the metabolism of similar highly studied compounds, such as benzalkonium chloride, does not lead to rapid degradation in mammals, both in animals of ecological interest, in livestock and pets, and in humans.

## Claims

1. Composition with virucidal activity **characterised in that** the active ingredient(s) is/are an aminium methyl sulfate selected from at least one of the following compounds:
i) 3-(Undec-10-enamido)-N,N, N-trimethyl-propan-1-aminium methyl sulfate 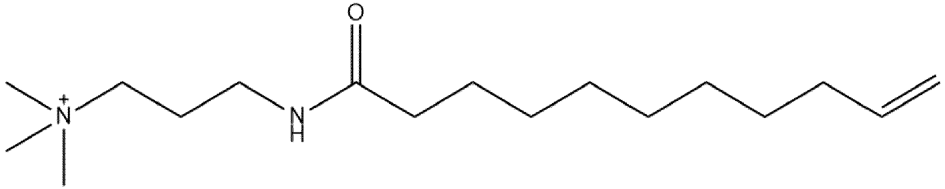 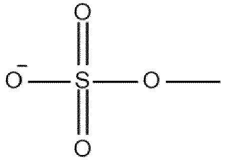
ii) 3-(Dodec-11-enamido)-N,N, N-trimethylpropan-1-aminium methyl sulfate 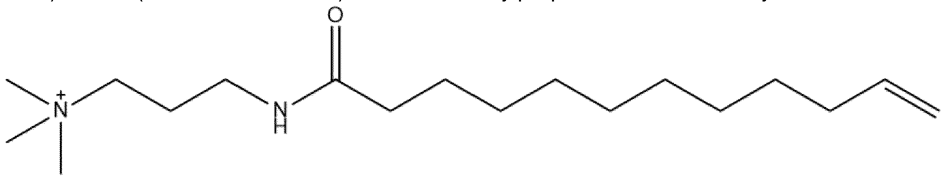 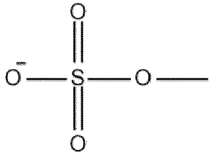
iii) 3-(Undec-10-enamido)-N-ethyl-N,N-dimethylpropan-1-aminium methyl sulfate 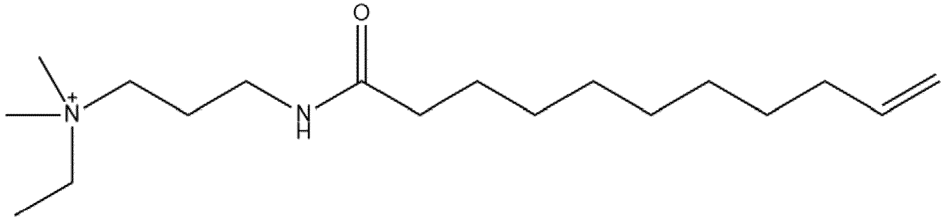 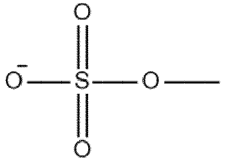
iv) 3-(Dodec-11-enamido)-N-ethyl-N,N-dimethylpropan-1-aminium methyl sulfate

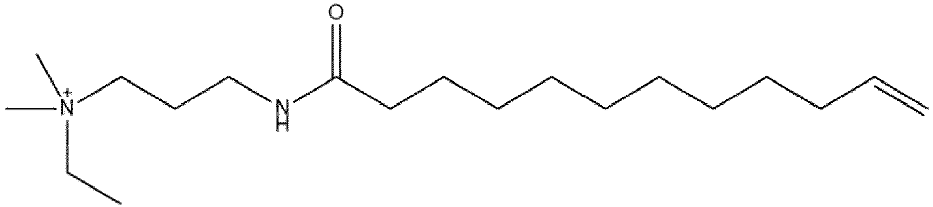

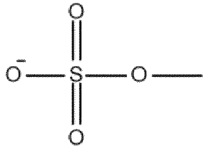

2. Composition with virucidal activity according to claim 1, **characterised in that** the claimed compounds are in a concentration of 0.01% to 5% w/w in water.

3. Composition with virucidal activity according to claim 1, **characterised in that** the claimed compounds are in a concentration of 0.01% to 5% w/w in a 70% ethanol solution and the rest is propylene glycol.

4. Composition with virucidal activity according to claim 1, **characterised in that** it contains at least one of the claimed compounds and is active against lipid enveloped viruses and non-lipid enveloped viruses.

5. Composition with virucidal activity according to claim 4, **characterised in that** the concentration is from 0.1% to 0.3% w/w.

6. Composition with virucidal activity according to claim 1, **characterised in that** it is located inside a pressurised aerosol-generating container.

7. The use of the compounds according to claim 1, for the disinfection of viruses pathogenic to any animal or plant.

8. The use of the compounds according to claim 1, for the disinfection of viruses pathogenic to non-human mammalian animals.

9. The use of the compounds according to claim 1, for the disinfection of the following human viruses:
Adeno-associated virus, Aichi virus, Australian bat lyssavirus, Astrovirus MLB1, Astrovirus VA1, BK polyomavirus, Banna virus, Barmah forest virus, Bundingo ebolavirus,Bunyamwera virus, Bunyavirus La Crosse, Bunyavirus snowshoe hare, Cercopithecine herpesvirus, Chandipura virus, Chikungunya virus, Cosavirus A, Cowpox virus, Coxsackievirus, Crimean-Congo hemorrhagic fever virus, Dengue virus, Dhori virus, Dugbe virus, Duvenhage virus, Eastern equine encephalitis virus, Ebolavirus, Echovirus, Encephalomyocarditis virus, Epstein-Barr virus, European bat lyssavirus, GB virus C, Hepatitis G virus, Hantaan virus, Hendra virus, Hepatitis A virus, Hepatitis B virus, Hepatitis C virus, Hepatitis E virus, Hepatitis delta virus, Horsepox virus, Human adenovirus, Human astrovirus, Human bocavirus, Human bocavirus 2, Human coronavirus (including SARS-CoV-1, SARS-CoV-2, MERS, HKU1, E229, OC43, NL63), Human cosaviruses A, B, C, D, E, Human cytomegalovirus, Human enterovirus 68, Human enterovirus 70, Human herpesvirus 1, Human herpesvirus 2, Human herpesvirus 6, Human herpesvirus 7, Human herpesvirus 8, Human immunodeficiency virus, Human papillomavirus 1, Human papillomavirus 2, Human papillomavirus 16, Human papillomavirus 18, Human papillomavirus 116, Human parainfluenza, Human parvovirus 4, Human parvovirus B19, Human respiratory syncytial virus, Human rhinovirus, Human SARS coronavirus, Human spumaretrovirus, Human T-lymphotropic virus, Human torovirus, Influenza A virus, Influenza B virus, Influenza C virus, Influenza A(H1N1)pdm09 virus, Influenza A(H3N2) viruses, Influenza A(H5N1) virus Isfahan virus, JC polyomavirus, Japanese encephalitis virus, Junin arenavirus, KI Polyomavirus, Klassevirus, Kunjin virus, Lagos bat virus, Lake Victoria Marburgvirus, Langat virus, Lassa virus, Lordsdale virus, Louping ill virus, Lymphocytic choriomeningitis virus, Machupo virus, Mayaro virus, Measles virus, Melaka virus, Mengo encephalomyocarditis virus, Merkel cell polyomavirus, Mokola virus, Molluscum contagiosum virus, Monkeypox virus, Mumps virus, Murray valley encephalitis virus, New York virus, Nipah virus, Norwalk virus, O'nyong-nyong virus, Orf virus, Oropouche virus, Pichinde virus, Poliovirus, Punta toro phlebovirus, Puumala virus, Rabies virus, Rift valley fever virus, Rosavirus A, Ross river virus, Rotavirus A, Rotavirus B, Rotavirus C, Rubella virus, Sagiyama virus, Salivirus A, Sandfly fever Sicilian virus, Sapporo virus, Semliki forest virus, Seoul virus, Simian foamy virus, Simian virus 5, Sindbis virus, Southampton virus, St. louis encephalitis virus, Tick-borne powassan virus, Torque teno virus, Toscana virus, Uukuniemi virus, Vaccinia virus, Varicella-zoster virus, Variola virus, Venezuelan equine encephalitis virus, Vesicular stomatitis virus, Western equine encephalitis virus, WU polyomavirus, West Nile virus, Yaba monkey tumor virus, Yaba-like disease virus, Yellow fever virus, Zika virus.zz

10. The use of the compounds according to claim 9, for the disinfection of viruses belonging to the coronavirus, influenza (flu), poliovirus, adenovirus and norovirus families.

11. A method of disinfecting viruses on surfaces comprising applying solutions containing the compounds claimed in claim 1 to the surfaces for at least five minutes.

12. Use of the virucidal compounds according to claim 1, in medicine and veterinary medicine as antivirals, both with topical route and other routes of medical and veterinary use.
